# EUROPEAN PATENT APPLICATION

(11) **EP 1 113 271 A2**
(43) Date of publication of application: **04.07.2001**
(21) Application number: 00311047.5
(22) Date of filing: 12.12.2000
(51) Int. Cl.: G01N 33/545, G01N 33/544, G01N 33/552

(54) **Polymer-coated surfaces for adsorbing macromolecules and their use in analytical methods**

(30) Priority: 13.12.1999 GB 9929431
(71) Applicant: Ortho-Clinical Diagnostics, Amersham, Buckinghamshire HP7 OHJ (GB)
(72) Inventor: Summers, Malcolm Robert, Aylesbury, Buckinghamshire HP21 7LH (GB); Hall, Joanne Elizabeth, Aylesbury, Buckinghamshire HP20 1XH (GB)
(74) Representative: Mercer, Christopher Paul

(57) **Abstract**

Methods are described for coating a surface of an article with a polymer comprising monomers of p-xylylene, and/or ring halogenated p-xylylene, and/or p-fluoromethyl-xylylene and adsorbing macromolecules thereto. Reaction vessels comprising such polymer-coated surfaces and macromolecules adsorbed thereto are useful for adsorption and solid-phase separation of reactants in analytical methods.

## Description

### FIELD OF THE INVENTION

The present invention relates to articles, particularly reaction vessels, coated with polymers comprising monomers of p-xylylene, and/or ring halogenated p-xylylene, and/or p-fluoromethyl-xylylene that are capable of adsorbing macromolecules, and their use in analytical methods.

### BACKGROUND OF THE INVENTION

The adsorption of macromolecules (molecular mass greater than or equal to about 1 kilodalton) to surfaces finds utility in numerous applications including removing substances from water to render it potable, and concentrating substances onto the surface of a substrate in chromatographic and analytical methods.

The present invention finds particular utility in solid phase immunoassays. Immunoassays involve binding reactions between specific binding partners. One member of a pair of binding partners is generally referred to as the ligand and the other member is generally referred to as the receptor. Either member of a pair can be designated ligand or receptor. Many immunoassay formats are known and are widely used for the detection and measurement of various analytes. Solid phase immunoassays involve the separation of a labeled reagent that becomes bound to a solid surface from labeled reagent that is not bound to the solid surface. In an example of a competitive immunoassay, an analyte, often designated the ligand, competes with labeled analyte for binding to a limited amount of a receptor, which is generally immobilized or capable of being immobilized on a solid surface. In an example of an immunometric (sandwich) assay, a receptor, which is generally immobilized or capable of being immobilized on a solid surface, binds to a site of an analyte, and a labeled receptor binds to a separate site of the analyte. In both competitive and immunometric formats either the labeled reagent bound to the solid surface or the free labeled reagent can be detected as a measure of the presence or amount of the analyte.

The labeled reagent, which can be the labeled analyte, a labeled analogue of the analyte, a labeled ligand, or a labeled receptor, carries a detectable group. The detectable group can be a constituent part of the labeled reagent or it can be directly attached to the analyte, analogue of the analyte, ligand or receptor, for example by covalent bonding. The detectable group can also be indirectly attached to the analyte, analogue of the analyte, ligand or receptor before or during the assay procedure, for example via one or more additional receptors or ligands one of which carries the detectable group.

The macromolecule as used herein comprises a specific binding partner. Specific binding partners include, but are not limited to: antibody and antigen, antibody and anti-antibody, hapten and anti-hapten antibody, sugar and lectin, avidin and biotin, streptavidin and biotin, enzyme and cofactor, oligonucleotide probe and target nucleic acid. Analytes that can be determined using binding reaction methods include, but are not limited to, steroids, drugs, oligonucleotides, proteins, sugars, saccharides, oligo- and polysaccharides.

A "sample" as used herein, refers to any substance which may contain the analyte of interest. A sample can be biological fluid, such as whole blood or whole blood components including red blood cells, white blood cells, platelets, serum and plasma, ascites, urine, cerebrospinal fluid, and other constituents of the body which may contain the analyte of interest. Optionally, samples may be obtained from water, soil, and vegetation.

The detection of specific nucleic acid sequences using labeled-oligonucleotides in sandwich-type assays is well known. Labeled-oligonucleotides for use in assays based on nucleic acid amplification technology such as the polymerase chain reaction (PCR) enjoy particular utility. PCR and the application of labeled-oligonucleotides in PCR is described in numerous publications, for example, US Patent No. 5,229,297 to Schnipelsky et al., US Patent No. 5,328,825 to Warren et al., and US Patent No. 5,387,510 to Wu.

Solution-based analytical systems are extensively employed. A widely accepted alternative to a solution-based analytical system is a dry analytical element. Dry analytical elements and their use in analytical assays are described in numerous publications, including US Patent No. 4,258,001 to Pierce et al., US Patent No. 4,670,381 to Frickey et al., WO 82/2601 (published August 5, 1982), European Patent Application No. 051 183 (published May 12, 1982) and European Patent Application No. 066 648 (published December 15, 1982).

In heterogeneous immunoassay systems for determining a target analyte, either solution-based or in dry analytical elements, free labeled-immunoreactant (for example a labeled-analyte, a labeled analogue of an analyte, or a labeled receptor specific for an analyte) must be separated from bound labeled-immunoreactant. And, as stated above, either free or bound labeled-immunoreactant can be detected as a measure of the presence or amount of the target analyte.

Conventional labels include radioactive tags, enzymes, luminophores, chromophores, fluorophores, stable free radicals and enzyme cofactors, inhibitors and allosteric effectors. Detectable signal that is correlated with a target analyte can be obtained from measuring optical absorption, fluorescence, or luminescence as is well known.

The adsorption of proteins to surfaces of solid substrates such as, polystyrene, polypropylene, polyvinyl chloride, glass, nylon and nitrocellulose, has been widely exploited to bring about the partitioning of components in immunoassays. For example, US Patent No. 4,980,929 describes an injection molded synthetic resin carrier or substrate coated with immunologically-active material. Substrates comprising such immunologically-active material are useful for separating free from bound reactants in immunoassays.

The adsorption process depends upon a combination of interactive forces, including electrostatic and hydrophobic bonding. These forces are influenced by the properties of the surface, the substance that is to be bound to the surface, and the environment in which the substance is presented to the surface. Under ideal conditions adsorption can be essentially irreversible (Bagchi et al., *J Colloid Interface Sci,* 83 (1981) pp.460-478). In practice, desorption (disassociation) of a bound substance from a surface often constitutes a variable but significant proportion of the material initially adsorbed. Desorption occurs as a result of weak interactive forces between the surface and substance to be bound, which may be attributable to incompatibility between the substrate and the substance to be bound, or to unfavorable environmental conditions (e.g. pH or ionic strength). Other reasons include multi-layering of the substance on the surface (outer layers are less strongly adsorbed) or contamination of the surface. Surfaces can be contaminated from pollutants present in the atmosphere, handling, leaching of additives (lubricants, fillers, plasticisers) used in the preparation of the substrate, and the presence of unreacted monomers, mold release agents and machine oils (Zsom, *J Colloid Interface Sci,* 111 (1986) p 434).

In the context of immunoassays, immunoreactants, such as antigen, antibody or antibody fragment, may be immobilized on an insoluble substrate in order to capture a specific binding partner from a solution in contact with the substrate. For example, an antibody specific for human chorionic gonadotrophin (hCG) adsorbed to the walls of a polystyrene reaction vessel will sequester onto the reaction vessel walls hCG that is present in a liquid contained within the reaction vessel. The sequestered hCG can then be quantified by any of a variety of methods known to those skilled in the art. At any stage of the procedure desorption of immunoreactant from the solid phase into the liquid phase will alter the distribution between the two phases, resulting in erroneous determinations and increased assay variability.

Covalent attachment of substances to specific chemical groups of a substrate has been described as a means of reducing desorption (Quash et al., J. Immunol. Meth., 22 (1978) pp 165-174). However, it is believed that a large fraction of the material is adsorbed rather than covalently bonded (as much as 40-50%); desorption, therefore, remains problematic.

US Patent 4,980,299 discusses the adsorption of immunoreactants to the walls of "tubelets" constructed from polystyrene and other plastics. Much of the variability in the mass adsorbed is attributed to impurities at the surface of the plastic. The authors state that manufacture of the items using extremely pure monomer with minimal additives produces a carrier surface that can be uniformly coated with immunoreactant.

In many instances pure monomer cannot be used in the manufacture of a substrate (e.g. where filler compounds are required to color the substrate or to render it opaque). In other circumstances surface contamination from external sources may be unavoidable. In still other circumstances it may be desirable to manufacture the substrate from a material that is incompatible with adsorption or covalent coupling processes. In these cases adequate means for limiting desorption or the variability of adsorption has not been available.

### SUMMARY OF THE INVENTION

The problems noted above have been solved through the use of polymers, known generically as Parylenes, comprising monomers of p-xylylene, and/or ring halogenated p-xylylene, and/or p-fluoromethyl-xylylene. A polymer of the present invention present on a surface of a substrate is capable of adsorbing macromolecules thereto. Hence, the present invention involves a surface for performing an assay for an analyte wherein the surface has been coated with parylene and has a macromolecule adsorbed thereto, the macromolecule comprising a specific binding partner as defined herein.

Advantages of using polymer films of the present invention for the adsorption of biologically active compounds include: a) independence from the nature of the supporting substrate - undesirable surface contaminants from plastics or injection molding processes which may be exposed on the uncoated substrates are sealed beneath the polymer layer, b) supporting substrates can be manufactured from materials which are unsuitable for the attachment of biologically active compounds, and subsequent application of the polymer allows them to sustain the attachment of such compounds, c) the polymer films can be deposited *in vacuo* from a pure starting material, thus presenting a more uniform surface for the adsorption process than might be obtained using conventional surfaces, d) increased affinity for the adsorption of protein - adsorption of protein is higher, and desorption is lower on polymer-coated than on uncoated surfaces. The use of polymer-coated surfaces of the present invention results in improved and high quality analytical measurements that rely on surface-bound activity, such as immunoassays.

Substrates coated with polymers of the present invention can be expected to have advantages in many other applications where surface-bound bioactive molecules are used. These include membranes, and porous webs or surfaces used in biosensors. The transparency of the films lends them to applications in optical devices. The high strength of the films suggests that they could be deposited onto, and subsequently removed from, a support, allowing the coated film to be used without added bulk, or in contexts where extreme thinness or physical flexibility would be advantageous.

In general, any surface capable of accepting a polymer of the present invention can be used for any application in which it is desirable to adsorb a macromolecule.

As one object of the present invention an article is provided having a surface comprising a coating of a polymer consisting essentially of monomers of formula: 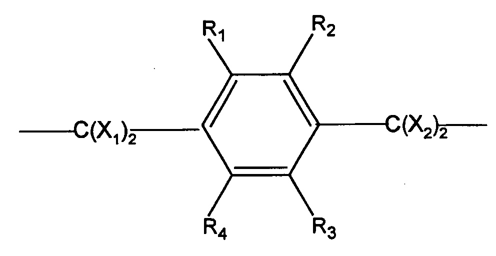 wherein R₁, R₂, R₃, and R₄ are independently hydrogen or halogen; X₁ and X₂ are independently hydrogen or fluorine, wherein the polymer coating comprises a macromolecule adsorbed thereto.

As another object of the present invention a method is provided for preparing an article having a polymer coating on a surface and a macromolecule adsorbed thereto comprising the steps of:
i) heating a compound of formula: 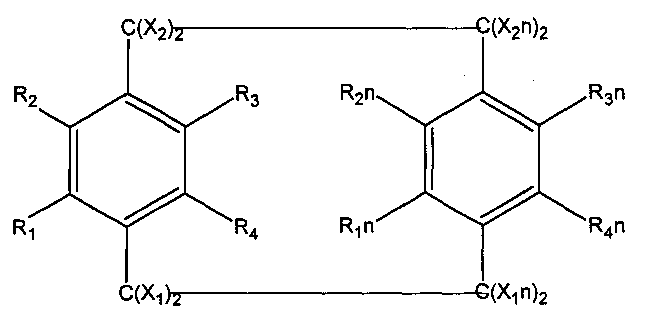 wherein R₁, R₂, R₃, R₄, R₁ₙ, R₂ₙ, R₃ₙ and R₄ₙ are independently hydrogen or halogen and X₁, X₁ₙ, X₂ and X₂ₙ are independently hydrogen or fluorine, to between about 150°C to about 200°C at a pressure between about 0.1 Torr to about 10 Torr, thereby forming a vapor;
ii) heating the vapor to between about 500°C to about 700°C at a pressure between about 0.001 Torr to about 10 Torr;
iii) cooling the vapor to between about 15°C to about 200°C deg at a pressure between about 0.001 Torr to about 10 Torr in the presence of said article, thereby forming a polymer coating on a surface of said article; and
iv) contacting the article of step iii) with the macromolecule.

The present invention finds particular utility in analytical devices, elements, kits and methods. Thus it is another object of the present invention to provide a method for determining the presence or amount of an analyte in a sample comprising the steps of:
i) contacting an article having a surface comprising a coating of a polymer consisting essentially of monomers of formula 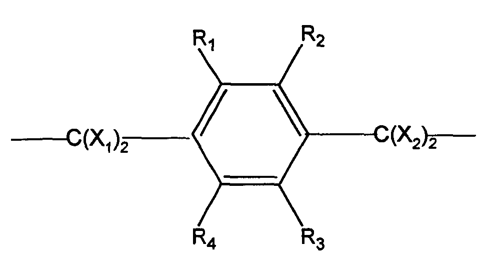 wherein R₁, R₂, R₃, and R₄ are independently hydrogen or halogen; X₁ and X₂ are independently hydrogen or fluorine, said polymer having a macromolecule adsorbed thereto, with
   A) the sample
   B) a labeled reagent, and
   optionally:
   1) one or more receptors specific for the analyte,
   2) one or more receptors specific for a ligand,
   3) one or more ligands
   4) the analyte or an analogue of the analyte, and
   wherein the labeled reagent is capable of binding directly to the macromolecule or indirectly to the macromolecule through a complex formed by the macromolecule, the analyte in the sample and any of 1), 2), 3) or 4) and wherein the amount of the labeled reagent that is bound is directly or inversely, linearly or non-linearly, proportional to the amount of analyte in the sample; and
   ii) detecting the labeled reagent that is directly or indirectly bound to the macromolecule or the labeled reagent that is not bound as a measure of the presence or amount of the analyte in the sample.

The macromolecule can be or comprise 1) one or more receptors specific for the analyte, 2) one or more receptors specific for a ligand, 3) one or more ligands, or 4) the analyte or an analogue of the analyte.

It is another object of the present invention to provide a method for a binding assay comprising the steps of contacting an article having a surface comprising a coating of a polymer consisting essentially of monomers of formula 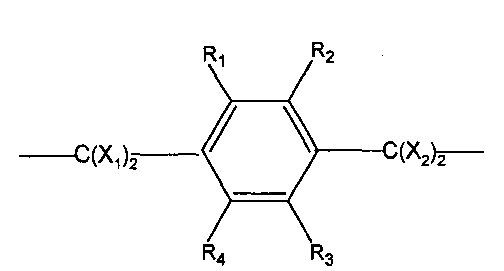 wherein R₁, R₂, R₃, and R₄ are independently hydrogen or halogen; X₁ and X₂ are independently hydrogen or fluorine, said polymer having a macromolecule adsorbed thereto, with
a sample suspected of containing an analyte, and detecting the analyte.

It is another object of the present invention to provide a method for determining the presence or amount of an analytein a sample comprising the steps of:
i) contacting an article having a surface comprising a coating of a polymer consisting essentially of monomers of formula 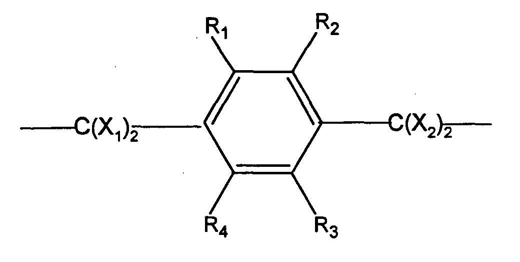 wherein R₁, R₂, R₃, and R₄ are independently hydrogen or halogen; X₁ and X₂ are independently hydrogen or fluorine, said polymer having a macromolecule adsorbed thereto, with
a) the sample,
b) a labeled analyte or labeled analogue of the analyte, or
c) a labeled receptor specific for the analyte,
wherein the macromolecule is a receptor specific for the analyte, or comprises the analyte, or comprises a receptor specific for the analyte, or comprises an analogue of the analyte; and
ii) separating the free labeled analyte or labeled analogue or labeled receptor from the bound labeled analyte or bound labeled analogue or bound labeled receptor; and
iii) detecting the free or the bound labeled analyte or labeled analogue of the analyte, or labeled receptor specific for the analyte as a measure of the presence or amount of the analyte in the sample.

In another aspect, a method is provided for determining the presence or amount of an analyte in a sample comprising the steps of:
i) contacting an article having a surface comprising a coating of a polymer as described above, the polymer-coated surface having a receptor specific for said analyte adsorbed thereto, with the sample;
ii) contacting the article with a labeled receptor specific for the analyte;
iii) separating the free labeled receptor from the bound labeled receptor; and
iv) detecting the free labeled receptor or the bound labeled receptor as a measure of the presence or amount of the analyte in the sample.

Yet another method is provided for determining the presence or amount of an analyte in a sample, comprising the steps of:
i) introducing a sample into a reaction vessel having therein a surface comprising a coating of a polymer as described above having a receptor specific for a ligand adsorbed thereto;
ii) introducing a receptor specific for the analyte into the reaction vessel, said receptor comprising the ligand bound thereto;
iii) introducing a labeled analyte or a labeled analogue of the analyte or a labeled receptor specific for the analyte into the reaction vessel;
iv) separating the free labeled analyte or the free labeled analogue or the free labeled receptor from the bound labeled analyte or bound labeled analogue or bound labeled receptor; and
v) detecting the free labeled analyte or the bound labeled analyte, or the free labeled analogue or the bound labeled analogue, or the free labeled receptor or the bound labeled receptor as a measure of the presence or amount of the analyte in the sample.

As yet another object of the invention a kit for determining the presence or amount of an analyte is provided comprising in the same or separate containers:
i) an article having a surface comprising a coating of a polymer as described above;
ii) a receptor specific for the analyte;
iii) a labeled analyte or a labeled analogue of the analyte or labeled receptor specific for the analyte; and
iv) reagents necessary for detecting the labeled analyte or the labeled analogue or the labeled receptor.

In yet another embodiment a kit is provided for determining the presence or amount of an analyte comprising in the same or separate containers:
i) an article having a surface comprising a coating of a polymer as described above;
ii) a receptor specific for a ligand;
iii) a receptor specific for the analyte, said receptor having the ligand bound thereto;
iv) a labeled analyte or a labeled analogue of the analyte or labeled receptor specific for the analyte; and
v) reagents necessary for detecting the labeled analyte or the labeled analogue or the labeled receptor.

As noted above, a polymer of the present invention consists essentially of monomers of formula 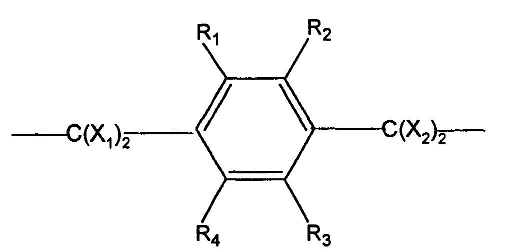 In a preferred embodiment, R1, R2, R3 and R4 are hydrogen, and X₁ and X₂ are hydrogen or X₁ and X₂ are fluorine; or R₁, R₂, R₃ and R₄ are halogen, and X₁ and X₂ are hydrogen or X₁ and X₂ are fluorine; or any one of R₁, R2, R₃ or R₄ is halogen and the other three are hydrogen, and X₁ and X₂ are hydrogen or X₁ and X₂ are fluorine; or any two of R₁, R₂, R₃ or R₄ are halogen and the other two are hydrogen, and X₁ and X₂ are hydrogen or X₁ and X₂ are fluorine; or any three of R₁, R₂, R₃ or R₄ are halogen and the other one is hydrogen, and X₁ and X₂ are hydrogen or X₁ and X₂ are fluorine.

In a more preferred embodiment R₁, R₂, R₃ and R₄, X₁ and X₂ are hydrogen; or any one of R₁, R₂, R₃ or R₄ is chlorine and the other three are hydrogen, and X₁ and X₂ are hydrogen; or any two of R₁, R₂, R₃ or R₄ is chlorine and the other two are hydrogen, and X₁ and X₂ are hydrogen.

The surface of an article to be coated with a polymer of the invention can comprise or consist essentially of polystyrene, polypropylene, polyvinyl chloride, glass, nylon or nitrocellulose. The thickness of the polymer coating is at least 0.2 microns, it is preferably between about 0.5 microns and about 20 microns and more preferably between about 5 microns and about 15 microns

The macromolecule adsorbed to the polymer can be a peptide, polypeptide, protein, glycoprotein, nucleic acid, oligonucleotide, saccharide, oligosaccharide, or derivative or combination thereof. It can be an analyte or analogue of an analyte or a receptor specific for an analyte. A preferred receptor for an analyte is an anti-analyte antibody. The macromolecule can be a receptor for a ligand other than an analyte. A preferred ligand is biotin, for which preferred receptors are avidin and strepavidin.

A preferred article for practicing the invention is a reaction vessel or an article capable of being formed into a reaction vessel. A preferred reaction vessel is a microwell.

### BRIEF DESCRIPTION OF THE DRAWINGS

FIG. 1 illustrates the adsorption of biotinylated bovine serum albumin to Parylene-coated and non-coated control microwells, by luminometric detection with streptavidin-HRP

FIG. 2 illustrates the desorption of streptavidin-HRP/biotinylated bovine serum albumin from Parylene-coated and non-coated control microwells.

FIG. 3 compares the binding of biotin to streptavidin bound to biotinylated bovine serum albumin adsorbed to Parylene-coated and non-coated control microwells.

FIG. 4 shows a dose-response curve of a competition assay for testosterone conducted using biotinylated anti-testosterone antibody adsorbed to streptavidin/biotinylated bovine serum albumin Parylene-coated microwells.

### DETAILED DESCRIPTION OF THE INVENTION

The presence of a polymer of the present invention on a substrate material improves its surface characteristics, and the polymer-coated surface is capable of adsorbing macromolecules thereto. In general, to prepare a polymer-coated surface of the invention, a substrate is exposed to a low-density vapor of a dimer which is pyrrolized to form two separate reactive diradicals resulting in condensation of polymer onto surfaces of the substrate. The polymer condensate or coating is extremely pure, chemically inert and pinhole-free; effectively isolating the original surface and any contaminants beneath it. The surface that receives the polymers of the invention can be particulate or non-particulate. A receiving surface can be composed of glass, nylon, polypropylene, polystyrene, poly(tetrafluoroethylene), polyvinyl chloride, nitrocellulose and the like. Particulate surfaces may be composed of the same materials noted, or they may be composed of aqueous-soluble or insoluble polymers, such as those described in US Patents 4,997,772; 5,147,777 and 5,149,737. The receiving surface may be chemically inert or have reactive functional groups.

The Parylenes, N, C and D and Nova HT: 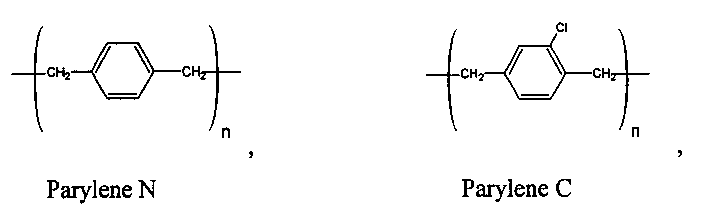 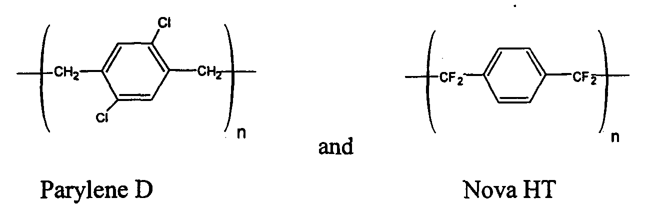 are available from Specialty Coating Systems, Indianapolis, Indiana. They are supplied as dimers, which are non-toxic white powders. Parylene coatings have been used to seal sensitive apparatus, such as electrical circuit boards, for use in harsh environments or to protect them from moisture (see US Patent 5,461,545). Parylene coatings have been applied to surgical implants (Tittman and Beach in Synthetic Biomedical Polymers: Concept and Application, Edited by Szycher and Robinson, pp 117-132. Technomic Publishing Co. Inc., Westport, Virginia, USA. (1980)) and they have been used to treat stents and catheters (US patents 5,873,904; 5,824,049 and 5,609,629 relate to stents, US patents 5,425,710 and 5,067,491 relate to catheters). They are biologically compatible (Eskin et al., *J. Biomed. Mater. Res.,* 10 (1976) pp 113-122) and have high lubricity. The use of Parylenes for the adsorption of bioactive substances in solid-phase separation-based immunoassays has not been described previously.

Methods for preparing Parylene coated surfaces are known. Briefly, such methods involve vaporization of a substituted or unsubstituted p-xylylene dimer and pyrrolization to form a diradical at elevated temperatures, and condensation of diradical at a lower temperature onto the surface of the article to be coated (see for example, US Patents 3,246,627; 3,301,707; 3,600,216 and EP 406,902). Dimers from which the polymer-coated surface can be prepared may be synthesized by well known methods, for example, see U.S. Patents 4,769,505; 4,806,702 and 5,110,903.

Polystyrene microwells are widely used in immunoassays as a combined reaction vessel and solid-phase separation device. The white microwells used in luminometric assays typically contain inorganic filler compounds and flow-enhancing and lubricating agents to facilitate the injection molding process. Surface-active and oily contaminants, which may be derived from such additives or from machine lubricants, are present on the surface of such microwells. The presence of these surface contaminants is associated with impaired assay performance.

It was determined that adsorbed immunoreactants desorb from the surface of these microwells into the liquid phase, and that desorption of immunoreactant adversely affects assay performance.

Microwells coated with Parylene polymer had improved characteristics with respect to protein adsorption, desorption, and biological activity in immunoassay compared with standard non-coated microwells.

Batches of white polystyrene microwells (Ortho-Clinical Diagnostics, Amersham, U.K.) contained in 96-well holding trays were coated with Parylenes C, N, and D. Film thickness was measured as 8, 12, and 15 microns, respectively. The internal capacity of the microwells is about 300 µL.

The examples that follow serve to illustrate the utility and advantages of the present invention and are not intended to limit its scope or spirit.

### Example 1

### Coating of Parylenes Onto a Substrate Surface

The process of coating Parylenes onto substrates is performed in a reactor (as described in commercial literature supplied by Speciality Coating Systems, Inc., 5707 West Minnesota Street, Indianapolis, IN 46241, USA and US Patent 3,246,627).

In the first stage, the dimer powder is vaporized by sublimation at 150° C at 1.0 torr. Further heating to 680° C at 0.5 torr converts the dimer to active monomer. Finally, the monomer vapor is cooled to 25° C at 0.1 Torr in the presence of the items to be coated, under which conditions it condenses and polymerises fully to form a cohesive film. Because they condense from the vapor phase, the polymer coatings conform exactly to the shape of the items treated. The monomer vapor penetrates and coats hidden surfaces (e.g. the inside of cylinders and tubes). Film thickness is controlled by the duration of exposure to monomer vapor. A coating thickness of at least about 0.2 microns is desirable. A thickness of about 0.5 microns to about 20 microns is preferred and a thickness of between about 5 microns to about 15 microns is most preferred.

### Example 2

### Protein Adsorption

Protein adsorption to Parylene-coated microwells and control microwells not coated with Parylene was assessed as follows:
A covalent conjugate of bovine serum albumin and biotin (B:BSA, Jackson
Immunoresearch Laboratories Inc., Pennsylvania, USA) at 6 µg/mL in 0.1 molar phosphate buffer, pH 7.0, was allowed to passively adsorb to Parylene-coated microwells and control microwells at ambient temperature (between about 20°C to about 25°C) for 10 minutes. The microwells were washed with 0.1 molar tris(hydroxymethyl)aminomethane buffer (Tris buffer), pH 8.5, containing 0.1% w/v bovine serum albumin. To each microwell was added, 210 µL of a solution of streptavidin in 0.1 molar phosphate buffer, pH 7.0, containing a covalent conjugate of horseradish peroxidase and streptavidin (SAV:HRP, Amersham International Plc., Amersham, U.K.) at one part per thousand (volume/volume) and incubated at ambient temperature, 20°C-25°C. During this time the streptavidin became bound to the biotin groups which had previously been adsorbed to the microwells via the B:BSA.

The microwells were washed with a solution containing 10 millimolar borate buffer, pH 8.4, prior to the addition of 200 µL of a luminogenic substrate for peroxidase (Vitros ECi

Signal Reagent; Ortho-Clinical Diagnostics, Amersham, U.K.). The Vitros ECi luminogenic substrate was used in all the experiments reported here.

Luminescence derived from the trace amount of SAV:HRP was measured and related to the mass of streptavidin bound to the microwells and thence to the mass of B:BSA adsorbed.

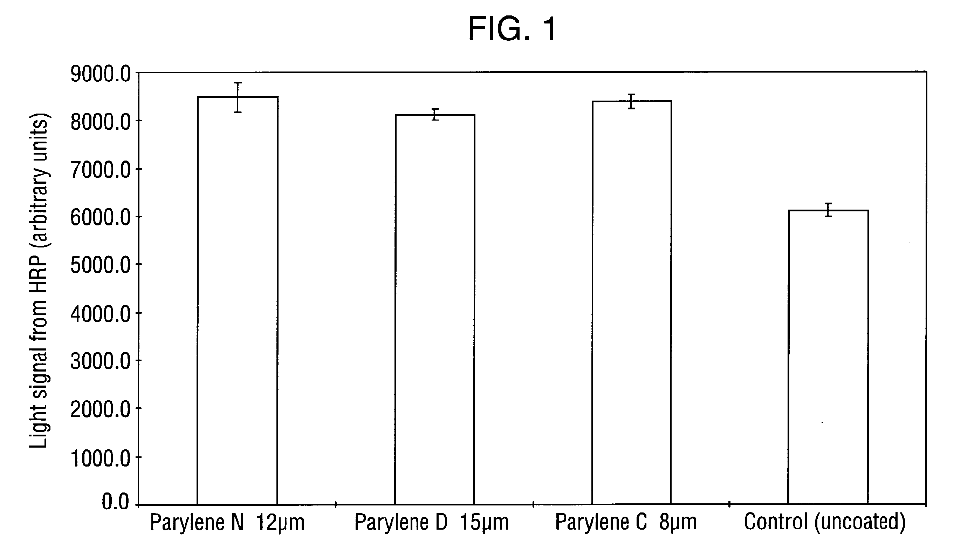

The Parylene-coated microwells demonstrated increased adsorption of B:BSA compared with the control microwells as illustrated by the greater average luminesence obtained from the Parylene-coated microwells as shown in FIG. 1 (the bars represent ± 1 standard deviation for 12 replicates).

### Example 3

### Protein Desorption

Protein desorption from Parylene-coated microwells and control microwells which were not coated with Parylene was assessed as follows:
B:BSA was allowed to passively adsorb to the microwells which were washed with Tris buffer containing BSA as in Example 2 above. To each microwell was then added 210 µL of a solution of streptavidin in 0.1 molar phosphate buffer, pH 7.0, and the microwells were incubated at ambient temperature. During this time the streptavidin became bound to the biotin groups of the B:BSA previously adsorbed to the microwells.

The microwells were washed twice with 0.1 molar Tris buffer, pH 8.5, and once with the same buffer supplemented with 50 grams per liter sucrose and 5 grams per liter sodium chloride. The microwells were aspirated, air dried and stored desiccated at 2-8°C before further use.

To each dried microwell was added 250 µL of 0.1 molar phosphate buffer, pH 7.0, and incubated at 37°C with agitation for one hour to allow any desorption to occur from the walls of the microwells. A 200 µL aliquot of liquid was removed from each microwell and transferred into microwells that had been pre-coated with rabbit anti-streptavidin antibody (a-SAV, Sigma-Aldrich Company Ltd., Dorset, U.K.). 200 µL aliquots from several different known concentrations of streptavidin in the same buffer were added to similar a-SAV coated microwells.

The a-SAV microwells were incubated at 37°C with agitation for one hour to allow streptavidin in solution to be bound by the solid-phase antibody. They were washed with a solution containing 10 millimolar borate buffer, pH 8.4.

To each a-SAV microwell was then added 200 µL of a solution of a covalent conjugate of horseradish peroxidase and biotin (B:HRP, Jackson Immunoresearch Laboratories Inc. Pennsylvania, USA) in 0.1 molar phosphate buffer, pH 7.0, containing 1% weight/volume BSA.

The a-SAV microwells were incubated at 37°C with agitation for 30 minutes to allow B:HRP to be bound via its biotin group to the streptavidin captured onto the solid phase via the solid-phase antibody.

The a-SAV microwells were then washed with a solution containing 10 millimolar borate buffer, pH 8.4, prior to the addition of 200 µL of luminogenic substrate. Luminescence was measured and related to the mass of peroxidase bound to the microwells, thence to the mass of streptavidin present, and finally to the mass of streptavidin desorbed from the microwells.

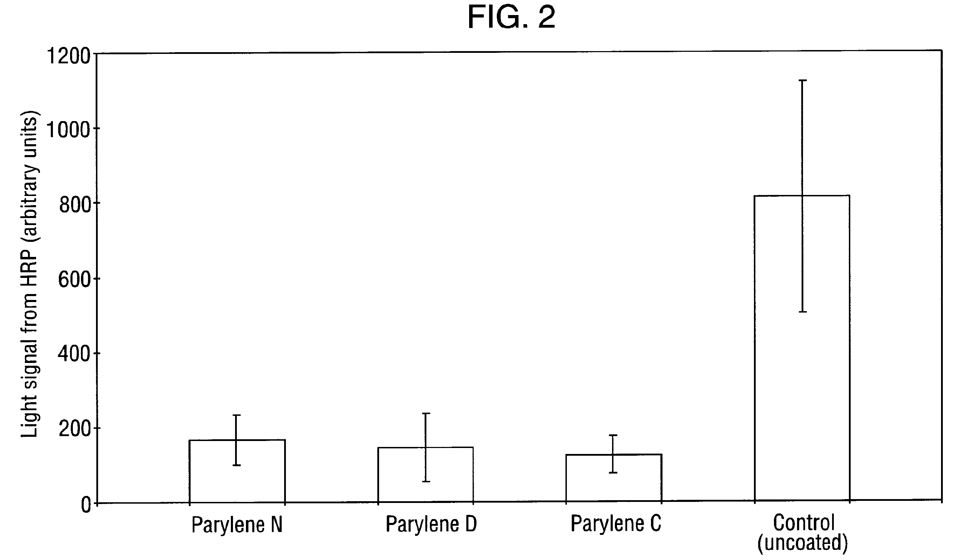

Parylene-coated microwells showed decreased desorption of protein compared with the control microwells as illustrated by the significantly smaller average lumininesence shown in FIG. 2 (the bars indicate ± 1 standard deviation for 12 replicates).

### Example 4

### Bio-activity of Immunoreactant Bound to Parylene-Coated Microwells

The bio-activity of Parylene-coated microwells and non-coated control microwells to which streptavidin had been bound via adsorbed B:BSA was assessed as follows:
Microwells were treated with B:BSA and streptavidin as in Example 3 above.

To each microwell was then added 200 µL of a solution containing 10 ng/mL d-biotin in 0.1 molar phosphate buffer, pH 7.0, containing one part in ten thousand (volume/volume) B:HRP and incubated at 37°C for 30 minutes with agitation. During this time the biotin and B:HRP bound to the streptavidin that was immobilized in the microwells.

The microwells were washed with a solution containing 10 millimolar borate buffer, pH 8.4, prior to the addition of 200 µL of luminogenic substrate for peroxidase.

Luminescence derived from the captured B:HRP was measured and related to the total mass of biotin bound by the microwells.

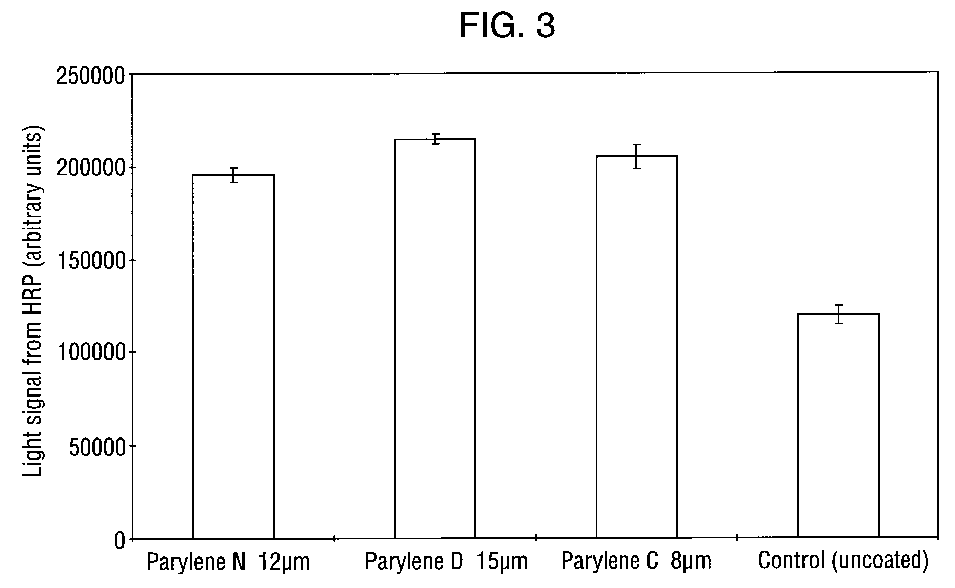

The Parylene-coated microwells demonstrated increased biotin-binding compared with the control microwells as determined by the significantly greater average luminesence obtained from the Parlyene coated microwells as shown in FIG. 3 (the bars indicate ± 1 standard deviation for 12 replicate measurements).

### Example 5

### Competitive-Format Immunoassay for Testosterone Using Parylene-Coated Microwells

The use of Parylene-coated microwells as a combined reaction vessel and separation device in an immunoassay was demonstrated as follows:

Parylene C-coated microwells were treated with B:BSA and streptavidin as in Examples 3 and 4 above. To one series of microwells was added 25 µL aliquots of human serum comprising known incremental amounts of the steroid hormone testosterone - the 'standards'. To a second series of microwells was added similar human serum samples containing testosterone in a quantity to be determined - the 'unknowns'. To each microwell was then added 100 µL of a solution containing a covalent conjugate of monoclonal anti-testosterone antibody and biotin (B:a-T, prepared by reaction of testosterone-specific murine immunoglobulin with a biotin n-hydroxysuccinimide ester derivative) in 50 millimolar phosphate buffer, pH 7.2. A limiting concentration of antibody was employed, that is, an amount less than that required to bind all the testosterone present in the reaction mixture.

The microwells were incubated at 37°C for 16 minutes to allow the B:a-T to bind a proportion of the testosterone present, and to allow the B:a-T to become bound to the microwell walls via the immobilized streptavidin.

To each microwell was then added 50 µL of a solution containing 60 ng/mL of a covalent conjugate of testosterone and horseradish peroxidase (T:HRP, prepared by reaction of testosterone carboxymethyl oxime with dicyclohexylcarbodiimide and n-hydroxysuccinimide followed by addition of HRP).

The microwells were incubated at 37°C for an additional 16 minutes to allow binding to continue, and then washed with a solution containing 10 millimolar borate buffer, pH 8.4, prior to the addition of 200 µL of luminogenic substrate for peroxidase.

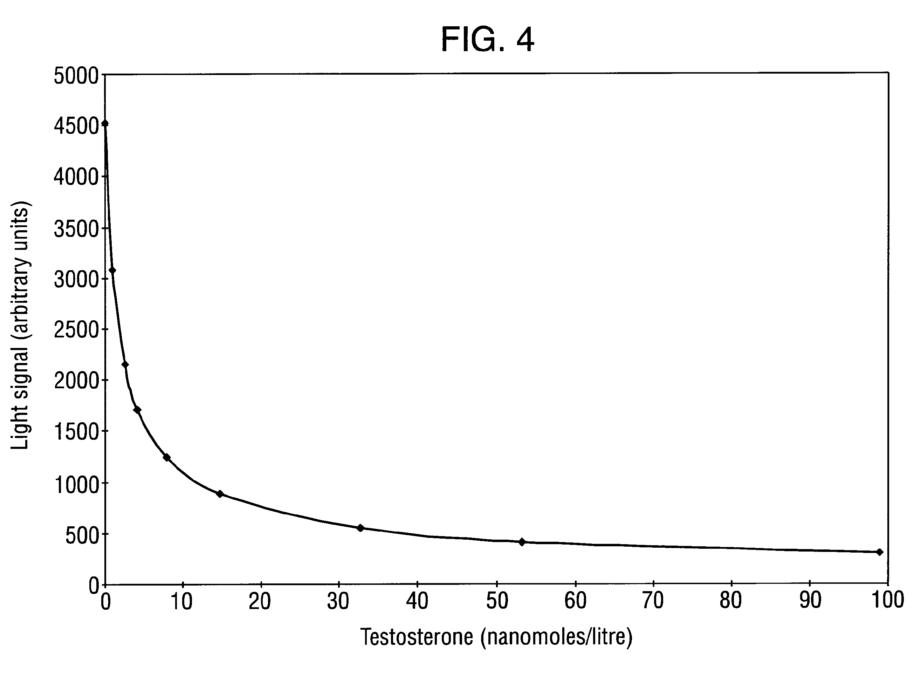

Luminescence was measured and related to the mass of testosterone in the standards as shown in the graph of FIG. 4. The data points in FIG. 4 represent the standards. A mathematical relation between the measured signal and amount of testosterone was derived using residual least-squares curve-fitting methodology (curve in FIG. 4). The amount of testosterone in the unknowns was calculated from the mathematical relation and the measured luminescence of the unknowns. Several replicate measurements were made. The calculated mean levels of testosterone, and the respective coefficients of variation about the mean are shown in the following table:

| Unknown | Mean light signal (arbitrary units) | Testosterone (nanomoles/L) | Coefficient of Variation (%) | Number of Replicates |
|---|---|---|---|---|
| 1 | 1937 | 3.34 | 3.3 | 4 |
| 2 | 935 | 13.06 | 3.7 | 5 |
| 3 | 512 | 36.72 | 2.8 | 5 |

The Parylene-coated microwells provided a sensitive and robust assay. Due to its reliance on precisely-controlled limiting concentrations of antibodies, this type of competition assay is particularly vulnerable to imprecision and inaccuracy arising from desorption of immunoreactants from the solid-phase. Typically, such imprecision is characterized by high variability within replicate measurements. The low variability as reflected in the small coefficients of variation implies that desorption of immunoreactants from the Parylene-coated microwells was of a minor order.

The invention has been described in detail with respect to particular preferred embodiments. It will be understood that variations and modifications can be effected without departing from the scope and spirit of the invention. The entire contents of all cited patents, patent applications, and non-patent disclosures are expressly incorporated herein by reference.

## Claims

1. An article having a surface comprising a coating of a polymer consisting essentially of monomers of formula: 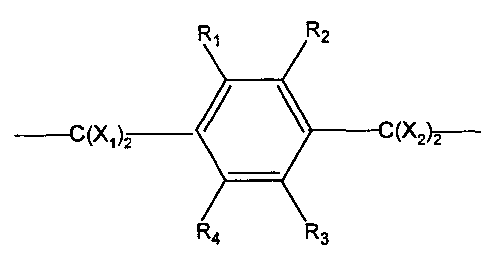 wherein R₁, R₂, R₃, and R₄ are independently hydrogen or halogen; X₁ and X₂ are independently hydrogen or fluorine, having a macromolecule adsorbed thereto.

2. The article of claim 1 wherein R₁, R₂, R₃ and R₄ are hydrogen, and X₁ and X₂ are hydrogen or X₁ and X₂ are fluorine; or R₁, R₂, R₃ and R₄ are halogen, and X₁ and X₂ are hydrogen or X₁ and X₂ are fluorine; or any one of R₁, R₂, R₃ or R₄ is halogen and the other three are hydrogen, and X₁ and X₂ are hydrogen or X₁ and X₂ are fluorine; or any two of R₁, R₂, R₃ or R₄ are halogen and the other two are hydrogen, and X₁ and X₂ are hydrogen or X₁ and X₂ are fluorine; or any three of R₁, R₂, R₃ or R₄ are halogen and the other one is hydrogen, and X₁ and X₂ are hydrogen or X₁ and X₂ are fluorine.

3. The article of claim 1 wherein R₁, R₂, R₃ and R₄, X₁ and X₂ are hydrogen; or any one of R₁, R₂, R₃ or R₄ is chlorine and the other three are hydrogen, and X₁ and X₂ are hydrogen; or any two of R₁, R₂, R₃ or R₄ is chlorine and the other two are hydrogen, and X₁ and X₂ are hydrogen.

4. The article of claim 1 wherein the surface consists essentially of polystyrene, polypropylene, polyvinyl chloride, glass, nylon or nitrocellulose.

5. The article of claim 1 wherein the thickness of the polymer coating is at least 0.2 microns.

6. The article of claim 1 wherein the thickness of the polymer coating is in the range between about 0.5 microns and about 20 microns.

7. The article of claim 1 wherein the thickness of the polymer coating is in the range between about 5 microns and about 15 microns.

8. The article of claim 1 wherein the macromolecule is a peptide, polypeptide, protein, glycoprotein, nucleic acid, oligonucleotide, saccharide, oligosaccharide, or derivative or combination thereof.

9. The article of claim 1 wherein the macromolecule is a receptor.

10. The article of claim 1 wherein the macromolecule is an analyte or analogue of an analyte.

11. The article of claim 1 wherein the macromolecule is a receptor for a ligand.

12. The article of claim 11 wherein the ligand is biotin.

13. The article of claim 1 wherein the article is a reaction vessel or capable of being formed into a reaction vessel.

14. The reaction vessel of claim 13 wherein the reaction vessel is a microwell.

15. The reaction vessel of claim 13 wherein R₁, R₂, R₃ and R₄ are hydrogen, and X₁ and X₂ are hydrogen or X₁ and X₂ are fluorine; or R₁, R₂, R₃ and R₄ are halogen, and X₁ and X₂ are hydrogen or X₁ and X₂ are fluorine; or any one of R₁, R₂, R₃ or R₄ is halogen and the other three are hydrogen, and X₁ and X₂ are hydrogen or X₁ and X₂ are fluorine; or any two of R₁, R₂, R₃ or R₄ are halogen and the other two are hydrogen, and X₁ and X₂ are hydrogen or X₁ and X₂ are fluorine; or any three of R₁, R₂, R₃ or R₄ are halogen and the other one is hydrogen, and X₁ and X₂ are hydrogen or X₁ and X₂ are fluorine.

16. The reaction vessel of claim 13 wherein R₁, R₂, R₃ and R₄, X₁ and X₂ are hydrogen; or any one of R₁, R₂, R₃ or R₄ is chlorine and the other three are hydrogen, and X₁ and X₂ are hydrogen; or any two of R₁, R₂, R₃ or R₄ is chlorine and the other two are hydrogen, and X₁ and X₂ are hydrogen.

17. The reaction vessel of claim 13 wherein the thickness of the polymer coating is at least 0.2 microns.

18. The reaction vessel of claim 13 wherein the thickness of the polymer coating is in the range between about 0.5 microns and about 20 microns.

19. The reaction vessel of claim 13 wherein the thickness of the polymer coating is in the range between about 5 microns and about 15 microns.

20. The article of claim 1 wherein the surface is particulate.

21. The article of claim 20 wherein the particles are water-insoluble.

22. A method for a binding assay comprising the steps of:
i) providing an article having a surface comprising a coating of a polymer consisting essentially of monomers of formula 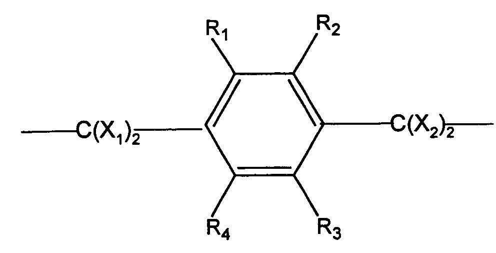 wherein R₁, R₂, R₃, and R₄ are independently hydrogen or halogen; X₁ and X₂ are independently hydrogen or fluorine, said polymer having a macromolecule adsorbed thereto,
ii) contacting said article with a sample suspected of containing an analyte, and
iii) detecting the analyte.

23. The method of claim 22 wherein the macromolecule is one or more of:
1) one or more receptors specific for the analyte,
2) one or more receptors specific for a ligand,
3) one or more ligands, or
4) the analyte or an analogue of the analyte.

24. The method of claim 22 wherein the article is a reaction vessel or capable of being formed into a reaction vessel.

25. The method of claim 23 wherein the reaction vessel is a microwell.

26. The method of claim 22 wherein R₁, R₂, R₃ and R₄ are hydrogen, and X₁ and X₂ are hydrogen or X₁ and X₂ are fluorine; or R₁, R₂, R₃ and R₄ are halogen, and X₁ and X₂ are hydrogen or X₁ and X₂ are fluorine; or any one of R₁, R₂, R₃ or R₄ is halogen and the other three are hydrogen, and X₁ and X₂ are hydrogen or X₁ and X₂ are fluorine; or any two of R₁, R₂, R₃ or R₄ are halogen and the other two are hydrogen, and X₁ and X₂ are hydrogen or X₁ and X₂ are fluorine; or any three of R₁, R₂, R₃ or R₄ are halogen and the other one is hydrogen, and X₁ and X₂ are hydrogen or X₁ and X₂ are fluorine.

27. The method of claim 22 wherein R₁, R₂, R₃ and R₄, X₁ and X₂ are hydrogen; or any one of R₁, R₂, R₃ or R₄ is chlorine and the other three are hydrogen, and X₁ and X₂ are hydrogen; or any two of R₁, R₂, R₃ or R₄ is chlorine and the other two are hydrogen, and X₁ and X₂ are hydrogen.

28. The method of claim 22 wherein the thickness of the polymer coating is at least 0.2 microns.

29. The method of claim 22 wherein the thickness of the polymer coating is in the range between about 0.5 microns and about 20 microns.

30. The method of claim 22 wherein the thickness of the polymer coating is in the range between about 5 microns and about 15 microns.

31. A method for preparing an article having a polymer coating on a surface and a macromolecule adsorbed thereto comprising the steps of:
i) heating a compound of formula: 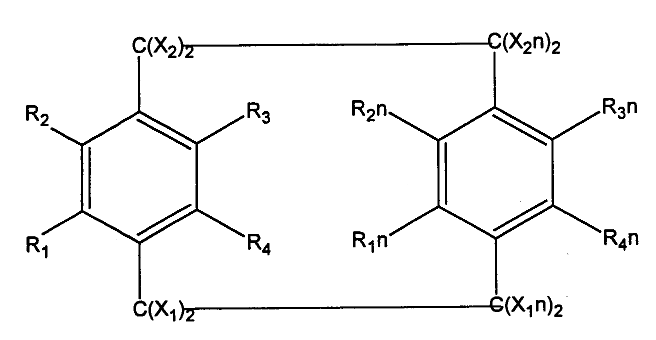 wherein R₁, R₂, R₃, R₄, R₁ₙ, R₂ₙ, R₃ₙ and R₄ₙ are independently hydrogen or halogen and X₁, X₁ₙ, X₂ and X₂ₙ are independently hydrogen or fluorine, to between about 150°C to about 200°C at a pressure between about 0.1 Torr to about 10 Torr, thereby forming a vapor;
ii) heating the vapor to between about 500°C to about 700°C at a pressure between about 0.001 Torr to about 10 Torr;
iii) cooling the vapor to between about 15°C to about 200°C deg at a pressure between about 0.001 Torr to about 10 Torr in the presence of said article, thereby forming a polymer coating on a surface of said article; and
iv) contacting the article of step iii) with the macromolecule.

32. A kit for determining the presence or amount of an analyte comprising in the same or separate containers:
i) an article having a surface comprising a coating of a polymer consisting essentially of monomers of formula: 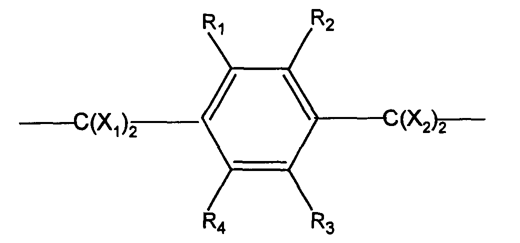 wherein R₁, R₂, R₃, and R₄ are independently hydrogen or halogen; X₁ and X₂ are independently hydrogen or fluorine;
ii) a receptor specific for the analyte;
iii) a labeled analyte or a labeled analogue of the analyte or a labeled receptor specific for the analyte; and
iv) reagents necessary for detecting the labeled analyte or the labeled analogue or the labeled receptor.

33. A kit for determining the presence or amount of an analyte comprising in the same or separate containers:
i) an article having a surface comprising a coating of a polymer consisting essentially of monomers of formula: 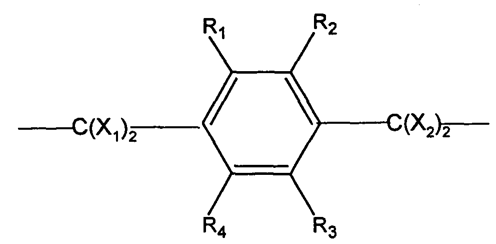 wherein R₁, R₂, R₃, and R₄ are independently hydrogen or halogen; X₁ and X₂ are independently hydrogen or fluorine;
ii) a receptor specific for a ligand;
iii) a receptor specific for the analyte, said receptor having the ligand bound thereto;
iv) a labeled analyte or a labeled analogue of the analyte or a labeled receptor specific for the analyte; and
v) reagents necessary for detecting the labeled analyte or the labeled analogue or the labeled receptor.
